# EUROPEAN PATENT APPLICATION

(11) **EP 1 149 844 A2**
(43) Date of publication of application: **31.10.2001**
(21) Application number: 01303411.1
(22) Date of filing: 11.04.2001
(51) Int. Cl.: C07K 14/46, C07K 14/47, C12N 15/06

(54) **Truncated reelin protein and DNA encoding the same**

(30) Priority: 11.04.2000 JP 2000109954
(71) Applicant: Riken, Wako-shi, Saitama 351-0198 (JP)
(72) Inventor: Mikoshiba, Katsuhiko, Mitaka-shi, Tokyo 181-0001 (JP); Tabata, Hidenori, Chiba-shi, Chiba 262-0032 (JP); Nakajima, Kazunori, Itabashi-ku, Tokyo 174-0063 (JP)
(74) Representative: Brasnett, Adrian Hugh

(57) **Abstract**

The present invention provides a truncated Reelin protein containing a F-spondin domain and a CR-50 recognition site but containing no repeat site of a Reelin protein, and a DNA encoding the truncated Reelin protein. The truncated Reelin protein and the DNA encoding this protein of the present invention can be utilized for treatment of diseases including agyria due to abnormal alignment of neurons.

## Description

### FIELD OF THE INVENTION

The present invention relates to a truncated isoform of a Reelin protein and a DNA encoding the truncated Reelin protein.

### BACKGROUND OF THE INVENTION

Neuroblasts born on the cerebral ventricle side migrate to their destinations following this developmental fate and achieve their final differentiation in the developmental stages of the central nerve system. The question is how neuroblasts recognize their destinations. Reeler mice have been long known as mutant mice which are very useful in studying this mechanism. In Reeler mutant mice, neuroblasts cannot migrate to their correct destinations, so that abnormal alignment of neuroblasts are found in various locations in the central nerve system including cerebrum, cerebellum, and hippocampus.

To identify a molecule deficient in a Reeler mutant mouse, we immunized a Reeler mutant mouse with a normal embryonic mouse brain, obtaining a monoclonal antibody CR-50 (Neuron 14, 899-912, 1995). Therefore, localized expression of CR-50 antigens in Cajal Retzius cells was found in cerebrum.

Then, a Reelin protein which is a causative gene of the Reeler mutant was cloned (Nature 374, 719-723, 1995, Genomics 26, 543-549, 1995, Nature Genetics 10, 77-82, 1995).

A Reelin protein is a huge extracellular protein, comprising a F-spondin domain having a certain degree of homology with F-spondin on its N-terminal side, and of eight Reelin repeats occupying the most of Reelin protein on its C-terminal side beyond the hinge region (Nature 374, 719-723, 1995). Each Reelin repeat contains an EGF-like motif so that Reelin protein is predicted to have features of extracellular matrix (ECM).

In addition, it was shown that a CR-50 antigen is a Reelin protein itself and can also inhibit the function of Reelin both in vitro and in vivo (J. Neurosci., 17, 23-31, 1997; Nature 385, 70-74, 1997; J. Neurosci., 17, 3599-3609, 1997; Proc. Natul. Acad. Sci. USA, 94, 8196-8201, 1997).

Furthermore, the recognition site of CR-50 is confirmed to be placed between a F-spondin domain and a Reelin repeat I (J. Neurosci., 17, 23-31, 1997).

Based on the above background, the N-terminal of Reelin is a site directly involved in Reelin function. Moreover, Reelin repeat is thought to localize Reelin proteins as ECM-like molecules in the vicinity of Reelin-producing cells, such as Cajal-Retzius cells, so as to give more localized signals.

### SUMMARY OF THE INVENTION

An object of the present invention is to confirm the presence of a Reelin (herein after referred as "Reelin") protein in Amphibian whose brain is normally shown to have no laminated structure.

The inventors cloned Reelin homologous molecules of Xenopus and confirmed the presence of a truncated isoform produced by alternative splicing during the process. This isoform has a F-spondin domain and a CR-50 recognition site, but has no Reelin repeat (hereinafter referred to as "repeat site."). Moreover, the inventors showed that this type of isoform is also present in a mouse. Since these isoforms have no repeat site, it is thought that they have no properties of an ECM-like molecule but play a role as humoral factors distributed distantly. The inventors completed this invention based on these understandings.

The present invention is summarized as follows.
(1) A truncated Reelin protein comprising an F-spondin domain and a CR-50 recognition site of a Reelin protein but containing no repeat site.
(2) The truncated Reelin protein of (1) which is derived from Xenopus or mice.
(3) The truncated Reelin protein of (2) which is either one of the following proteins (a) or (b):
   (a) a protein consisting of an amino acid sequence represented by SEQ ID NO: 2,
   (b) a protein consisting of an amino acid sequence differing from the amino acid sequence of SEQ ID NO: 2 by deletion, substitution, or addition of one or more amino acids, and having Reelin protein activity.
(4) The truncated Reelin protein of (2) which is either one of the following proteins (a) or (b):
   (a) a protein consisting of an amino acid sequence represented by SEQ ID NO: 4,
   (b) a protein consisting of an amino acid sequence differing from the amino acid sequence of SEQ ID NO: 4 by deletion, substitution, or addition of one or more amino acids, and having Reelin protein activity.
(5) A DNA encoding a truncated Reelin protein comprising an F-spondin domain and a CR-50 recognition site of a Reelin protein but contains no repeat site.
(6) The DNA of (5) which is derived from Xenopus or mice.
(7) The DNA of (6) encoding a truncated Reelin protein which is either one of the following proteins (a) or (b):
   (a) a protein consisting of an amino acid sequence represented by SEQ ID NO: 2,
   (b) a protein consisting of an amino acid sequence differing from the amino acid sequence of SEQ ID NO: 2 by deletion, substitution, or addition of one or more amino acids, and having Reelin protein activity.
(8) The DNA of (7), which is any one of the following DNAs (a) to (c):
   (a) a DNA having a nucleotide sequence represented by SEQ ID NO: 1,
   (b) a DNA hybridizing to a nucleic acid probe which comprises a sequence of 1456^{th} to 2273^{rd} nucleotide in the nucleotide sequence of SEQ ID NO: 1 under stringent conditions [for example, at 42°C in the presence of 5 x SSPE and 50% formamide (20 x SSPE = 3 M NaCl, 173 mM NaH₂PO₄, 25 mM EDTA)], and encoding a protein having Reelin protein activity.
   (c) a DNA having a nucleotide sequence which is a degenerate sequence of that of the DNA (a) or (b).
(9) The DNA of (6) encoding either one of the following truncated Reelin proteins (a) or (b):
   (a)A protein comprising an amino acid sequence represented by SEQ ID NO: 4,
   (b) a protein comprising an amino acid sequence differing from the amino acid sequence of SEQ ID NO: 4 by deletion, substitution, or addition of one or more amino acids, and having Reelin protein activity.
(10) The DNA of (9) which is any one of the following DNAs (a) to (c):
   (a) a DNA having a nucleotide sequence represented by SEQ ID NO: 3,
   (b) A DNA hybridizing to a nucleic acid probe which comprises a sequence of 2053^{rd} to 2758^{th} nucleotides in the nucleotide sequence of SEQ ID NO: 3 under stringent conditions [for example, at 42°C in the presence of 5 x SSPE and 50% formamide (20 x SSPE = 3 M NaCl, 173 mM NaH₂PO₄, 25 mM EDTA)], and encoding a protein having Reelin protein activity, or
   (c) A DNA having a nucleotide sequence which is a degenerate sequence of that of the DNA (a) or (b).

In this specification the term "Reelin protein" means a causative gene of Reeler mutant mice and an extracellular matrix protein comprising a signal peptide, F-spondin domain, CR-50 recognition site, and Reelin repeat.

The term "F-spondin domain" is a region that is shown to have homology with F-spondin on the N-terminal side of a Reelin protein.

The term "CR-50 recognition site" is a site that a CR-50 antibody recognizes in a mouse Reelin protein, and a region homologous to the CR-50 recognition site of mouse Reelin in a Reelin protein of other organisms. CR-50 antibodies can be prepared by the method described in Neuron 14, 899-912, 1995.

The term "repeat site" is a region on the C-terminal side of a Reelin protein that contains repeating units which consist of an amino acid sequence with an EGF-like motif at its center, and which are homologous to one other.

The term "Reelin protein activity" includes any biological and immunological action that a Reelin protein possesses. An example is the function of aligning neurons in their correct positions. This can be confirmed by the methods described in Nature 385, 70-74, 1997, J. Neurosci., 17, 3599-3609, 1997, and Proc. Natul. Acad. Sci. USA, 94, 8196-8201, 1997.

Reelin is an essential molecule in developing a normal laminated structure of cerebrum. Though no laminated structure is observed in Amphibian cerebrum, Xenopus Reelin was searched to confirm whether Reelin molecules are present in such an organism.

As a result of PCR using degenerate primers, PCR fragments of Xenopus Reelin (Xreelin) were obtained. Surprisingly, 3'-RACE analysis revealed the presence of a splicing mutant which contains most of the F-spondin domain and hinge region on the N-terminal side of Xreelin, but contains no repeat site. Expression of both an intact form of Xreelin and a truncated form of Xreelin started at the tail bud embryonic stage (st. 28) and continued until the end of the development into an adult. Northern blotting showed that a transcript of the intact Xreelin was about 12 kb, as in a mouse. On the other hand, a band of the truncated Xreelin was confirmed at a 3 to 4 kb-position. Moreover, transcripts of the intact Xreelin were detected in the primordiums of the olfactory bulb and optic tectum by in situ hybridization. At tadpole stage (st. 47), expression of the transcripts was also confirmed in the cerebellum. These results were almost identical to those for mice, but no expression was observed in the cerebrum. This is consistent with the fact that Xenopus brain has no laminated structure.

The truncated Reelin protein of this invention contains no Reelin repeat which accounts for approximately 85% of a Reelin protein. Reelin repeat is a region containing 8 repeats of a sequence having an EGF-like motif, suggesting that it binds with an extracellular matrix molecule having another EGF-like motif. Accordingly, Reelin repeat is a region required to localize Reelin proteins in extracellular matrix and a truncated isoform lacking this repeat site may diffuse farther in vivo.

For example, diseases including agyria, polymicrogyriam, and ectopic gray matter due to abnormal neuronal alignment can be treated by incorporating cDNA encoding truncated Reelin proteins of this invention into expression vectors, introducing the vectors into neuroblasts and nerve trunk cells and the like derived from the tissue of a patient, and transplanting those cells into the patient brain.

### SEQUENCE LISTING FREE TEXT

SEQ ID NOS: 5 to 17, 19, 20, and 22 to 28 show nucleotide sequences of primers.

SEQ ID NOS: 18 and 20 show nucleotide sequences of probes.

### BRIEF DESCRIPTION OF THE DRAWINGS

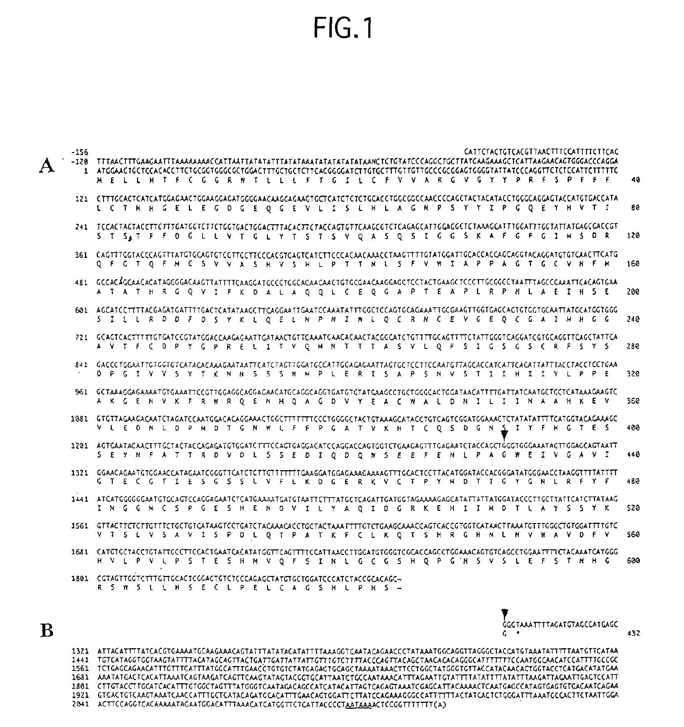

Figure 1 shows nucleotide sequences of the intact form of a Xenopus Reelin protein (Xreelin) (A) and of the truncated isoform (B) and a putative amino acid sequence of a Xenopus Reelin protein. "*" indicates a stop codon. Amino acids within a region pointed with an arrow (▼) are contained in the intact form of Reelin protein but not in the truncated isoform. A polyadenylation signal located 15 nucleotides upstream of a polyadenylation site is underlined.

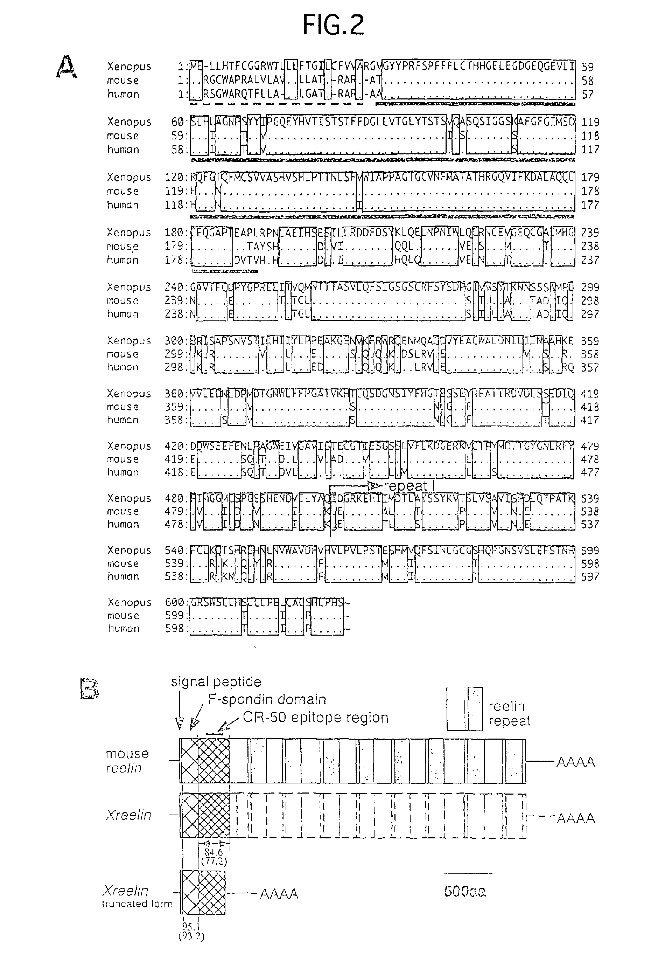

Figure 2 A shows multiple alignments of putative amino acids of Xenopus, mouse and human Reelin proteins. Putative signal peptides deduced based on Signalase software are underlined with broken lines. F-spondin domains are underlined with bold lines. Regions surrounded with frames contain amino acid residues common among all of the three types. Gaps inserted are shown with "-." Amino acid residues identical to those of Xreelin are shown with •.

Figure 2 B is a diagram showing a mouse Reelin protein, Xreelin and truncated Xreelin. Undefined regions are shown with a broken line. Regions enclosed with frames denote coding regions and lines denote noncoding regions.

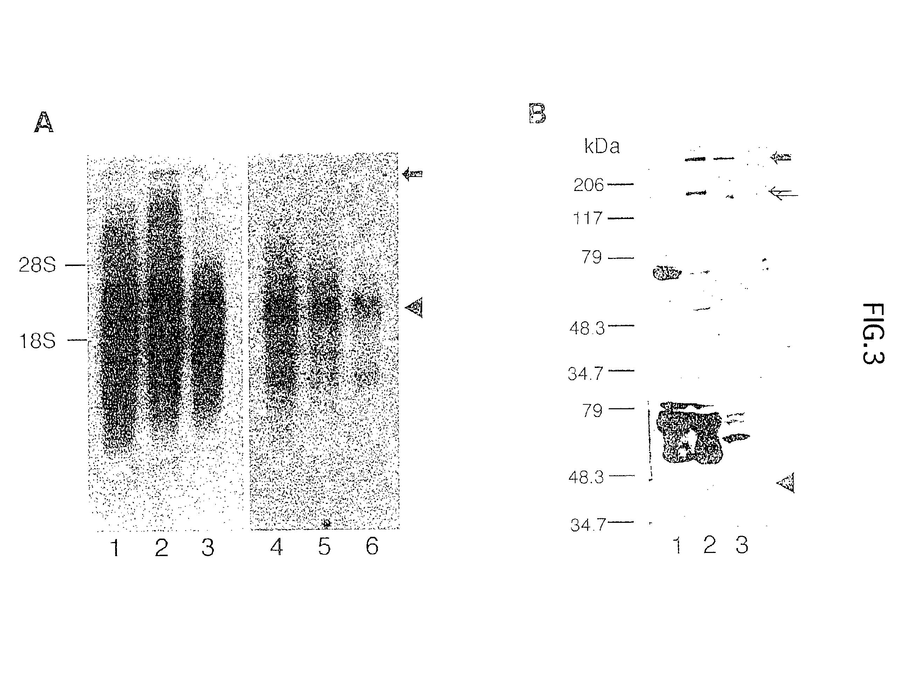

Figure 3A shows the results of Northern blot analysis on Xreelin. A bold arrow (←) indicates the intact form of a Reelin protein and an arrow ( 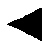 ) indicates the truncated form of a Reelin protein.

Figure 3B shows the results of Western blot analysis on Xreelin. A bold arrow (←) shows a Xreelin protein of the same size as a mouse Reelin protein detected with a Reelin protein antibody 142. A thin arrow (←) indicates a Xreelin protein fragment slightly smaller than a mouse Reelin protein fragment processed with metallo proteinase. An arrow ( 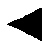 ) indicates a protein with a size predicted to be the truncated form of a Reelin protein.

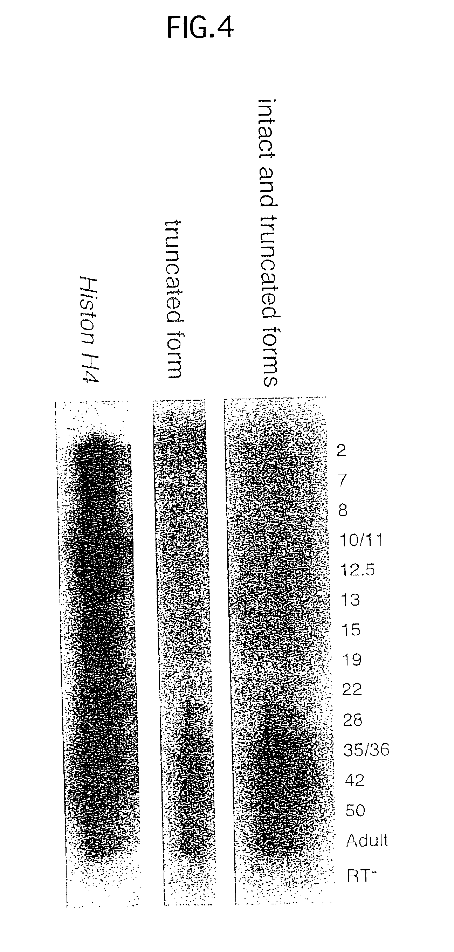

Figure 4 shows the results of RT-PCR using RNA samples at various developmental stages. At the top of a panel are numbers of developmental stages of samples in each lane.

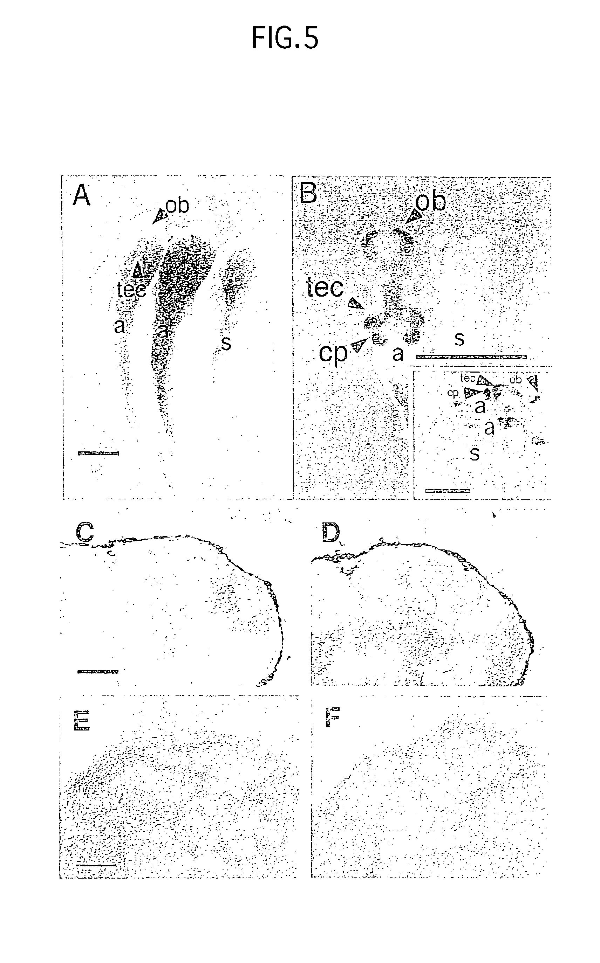

Figure 5 shows the distribution of the intact form of Xreelin mRNA by in situ hybridization (Fig. 5A, B, C, D, E, F). Anti-sense probes to XdII (D) and Eomesodermin (F) were also used to define the region of striatum in telencephalon and mitral cell in olfactory bulb. The specimens employed were (A) st. 35/36 whole embryos, (B) st. 47 whole brain, (E, F) horizontal sections of st. 51 olfactory bulb, and coronal sections of st. 54 at the level of (C, D) telencephalon, (G) tactum, and (H) spinal cord.
a: antisense probe, cp: cerebellum primordium, ob: olfactory bulb, s: sense probe, tec: tectum, Scale bars: A, B, 500 µm; C, D, G, H, 100 µm; E. F, 200 µm.

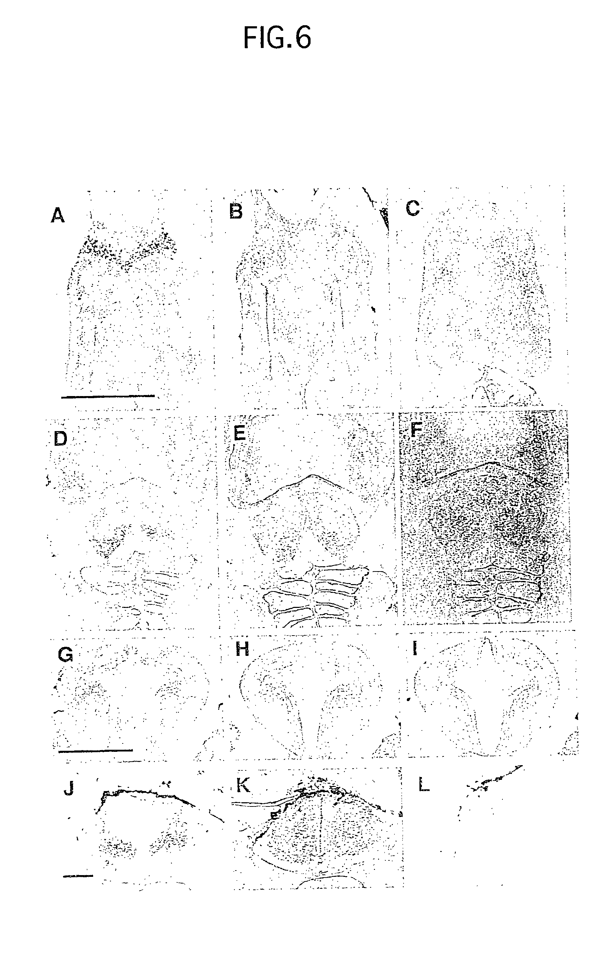

Figure 6 shows the comparison of expression pattern of the intact and truncated forms of Xreelin mRNA by in situ hybridization (A to C) and TaqMan PCR analysis (D). The intact (A) and truncated forms (B) of Xreelin mRNA in the horizontal section of st. 51 cerebellum were detected. The neighboring section was hybridized to sense probe of the intact form (C). Scale bars: 100 µm. The total RNA of seven parts of Xenopus brain (E) was subjected to TaqMan PCR analysis. Unfilled bars and filled bars indicate the copy number of intact and truncated forms, respectively.

### EXAMPLE

Now, a detailed description of the present invention will be given as follows. These examples are intended as an explanation, but do not limit the scope of the invention.

### Example 1 Cloning of Xenopus Reelin (Xreelin)

### Method

Random primed cDNA was synthesized from total RNA purified from stage (st.) 35 (Nieuwkoop, P. D. & Faber, J, 1967, in Normal Table of Xenopus laeis (Daudin), North-Holland Publishing Company, Amsterdam) Xenopus whole embryos using Super Script II (Gibco BRL), and subjected to PCR with degenerate primers. Using a set of degenerate primers [5'-A(A/G)TT(T/C)GGIAA(T/C)CA(A/G)TT(T/C)ATGTG-3' (SEQ ID NO; 5) and 5'-TG(T/C)TCICCCAT(T/C)CA(A/G)TT-3' (SEQ ID NO: 6)], the PCR fragment of Xreelin, which corresponds to 362 to 696 in the nucleotide sequence in Fig. 1 was obtained. To obtain the sequence further upstream from the PCR product, 5' RACE (5'-rapid amplification of cDNA end) was performed. Poly (A) + RNA was purified from the total RNA isolated from st. 35 Xenopus whole embryo using a Fast Track 2.0 kit (Invitrogen). The later procedures were carried out using Gibco 5' RACE system ver. 2 (GC rich protocol, Gibco BRL). First strand synthesis was primed with a gene specific primer [5'-ATGTCCTCACTGGAAAGATC-3' (SEQ ID NO: 7)]. The purified product of second strand reaction was A-tailed, and PCR performed thereon using the AUAP primer (Gibco BRL) and gene specific primer [5'-CAGCAACACATAGGGGACAA-3' (SEQ ID NO: 8)]. Moreover, 3' RACE (3'-rapid amplification of cDNA end) was conducted using 3' RACE system (Gibco BRL). First strand cDNA was generated by Super Script II with oligonucleotide [5'-GGCCACGCGTCGACTAGTACGAATTCATCTATAGC(T)₁₇-3' (SEQ ID NO: 9)] from total RNA of st. 35 Xenopus whole embryos. PCR was performed with an adapter primer, the sequence of which is the complement of the oligonucleotide described above without (T)₁₇ and a gene specific primer [5'-CAGTGTCGTTGCTTCCCACGTGAGTCATCTTCCCA-3' (SEQ ID NO: 10)]. This PCR product was further amplified with the adapter primer and a nested gene specific primer [5'-CGACAGGTACAGGATGTGTCAACTTCATGGCCACA-3' (SEQ ID NO: 11)]. A single band was obtained from this step, and was cloned into pGEM-T Easy vector (Promega). By sequencing this clone, a truncated isoform generated by alternative splicing was identified. The intact form specific sequence was elucidated by screening of the cDNA library. The Poly (A) + RNA prepared by Fast Track 2.0 kit from st. 56 Xenopus tadpole was employed to first strand synthesis in addition to the use of oligo(dT)₁₂₋₁₈ and random hexamers. The synthesized cDNA was ligated to λ ZapII phage vector (Stratagene). The 1 x 10⁶ independent clones were screened by a ³²P labeled probe corresponding to 414 to 1253 nucleotides in the nucleotide sequence in Fig. 1. Thus 2 clones were isolated and sequenced. Sequencing of these clones revealed the nucleotide sequence of the intact form (1294 to 1869). The nucleotide sequences shown in Fig. 1 were confirmed by at least 3 rounds of separate PCR reactions.

### Results

To obtain the Xenopus counterpart of Reelin, several sets of degenerate PCR primers were designed for various regions of the mouse Reelin. Among these primers, primer sets which enclose the boundary between the F-spondin domain and the CR-50 epitope region amplified a fragment with a sequence highly homologous to the mouse/human Reelin. Then 5' RACE and screening of a cDNA library were performed so that the 5'-non-coding region of 156 base pairs (bp) and the coding region of 1873 bp were isolated (Fig. 1A). These sequences were confirmed by sequencing three independent PCR products. Comparison of the amino acid sequence of Reelin between the lower vertebrate and mouse/human clarified the region conserved in evolution (D'Arcangelo, G., Miao, G.G., Shu-Cheng, C., Soares, H. D., Morgan, J. I. & Curran, T., 1995, Nature 374, 719-723; DeSilva, U., D' Arcangelo G., Braden, V., Chen, J., Miao, G., Curran, T. & Green, E. D., 1997, Genome res. 7, 157-164). The deduced amino acid sequence of Xreelin is conserved as between mouse/human Reelin over the extent of the sequenced region, but especially in the F-spondin domain. The identity and similarity within the F-spondin domain are estimated 93.2% and 95.1%, respectively, whereas the identity and similarity of the hinge region, which contains the CR-50 epitope region and is between the F-spondin domain and Reelin repeats, 77.2% and 84.6%, respectively (Figs. 2A and 2B). These results strongly suggest that the F-spondin domain is functionally important. This finding is particularly interesting as the inventors reported previously that CR-50, which recognizes just downstream of the F-spondin domain, blocks Reelin function both in vitro and in vivo (Ogawa, M., Miyata, T., Nakajima, K., Yagyu, K., Seike, M., Ikenaka, K., Yamamoto, H. & Mikoshiba, K., 19956, Neuron 14, 899-912; Del Rio, J. A., Heimrich, B., Borrell, V., Forster, E., Drakew, A., Alcantara S., Nakajima, K., Miyata, T., Ogawa, M., Mikoshiba, K., Derer, P., Frotscher, M. & Soriano, E., 1997, Nature 385, 70-74; Miyata, T., Nakajima, K., Mikoshiba, K. & Ogawa, M., 1997, J. Neurosci. 17, 3599-3609.; Nakajima, K., Mikoshiba, K., Miyata, T., Kudo, C. & Ogawa, M., 1997, Proc. Natl. Acad. Sci. USA 94, 8196-8201.

To obtain sequence information on downstream of the cloned region, 3'-RACE was performed. Thus, a truncated isoform of Xreelin containing only the F-spondin domain and the CR-50 epitope region was identified (Figs. 1B and 2B). Interestingly and surprisingly, this novel isoform does not have any Reelin repeat. On this truncated isoform, the nucleotide sequence differs from that of the intact form from the second nucleotide of the TGG codon. That is, the TGG is changed to TAA. This difference coverts the amino acid, tryptophan 433, to a stop codon TAA. Thereafter, a non-coding region of 698 bp follows, and poly-adenylation signal (AATAAA) appears at 15 bp upstream of the poly-adenylation site. The nucleotides that differ between the intact form and the truncated form correspond to the end of exon #11 of mouse Reelin (Royaux, I., Lambert de Rouvroit, C., D' Arcangelo, G., Demirov, D. & Goffinet, A. M., 1997, Genomics 46, 240-250). Analysis of how this isoform is generated is not yet complete but the most likely explanation is the skipping of splicing and reading into intron #11. PCR amplification using a forward primer to both isoforms and a reverse primer specific only to the truncated form was done on either genomic DNA or random primed cDNA as a template. Both gave an amplified product of the same size, which is compatible with the skipping of splicing mechanism.

### Example 2 Northern Blot Analysis

### Method

Five grams of poly (A) + RNA prepared from st. 50, 56, and 60 Xenopus tadpole head was applied on each lane, subjected to electrophoresis, and then transferred to Zeta-Probe Blotting membrane (Bio-Rad). 32 P labeled RNA probes were synthesized by in vitro transcription with [α-32P] UTP and [α-32P] CTP using the sequence common to the intact and truncated form, and the 3'-noncoding region of the truncated form which corresponds to nucleotides 414-1253 and 1302 - 2099, respectively. Hybridization was carried out in 5 x SSPE/50% formamide + 5 x Denhart's solution + 0.5% SDS at 60°C over night. The filter was washed twice in 2 x SSC + 0.1% SDS at 60°C for 30 minutes, and then in 0.1x SSC + 0.1% SDS at 60°C for 30 minutes.

### Results

To confirm the presence of this novel isoform, Northern blot analysis was performed (Fig. 3). Using an RNA probe to a sequence common to both forms, a transcript of 12 to 13 kb was detected. This result indicates that there is indeed an intact form of Reelin in Xenopus as a huge molecule like that found in mice. Apart from this transcript, a transcript of 2.8 kb was also found. Moreover, when the 3' non-coding region was used as a probe, only the shorter transcript of 2.8 kb was detected. The truncated form has a coding region of 1299 bases (b) and a 3' untranslated region of 698 bases, and , so far, 156 bases of the 5' untranslated region have been cloned and sequenced. Therefore, the mRNA of the truncated from is expected to consist of at least 2153 nucleotides. The size of the detected band on Northern blot analysis is larger than this size, but it is supposed that this discrepancy result from the unidentified region of the 5' terminal untranslated region. Thus, it is concluded that mRNA of the truncated form certainly exists.

### Example 3 Determination by RT-PCR of the time of Xreelin mRNA expression

### Method

Total RNA was prepared from 2 cell stage and stage 7, 8, 10/11, 12.5, 13, 15, 19, 22, 28, 35/36, 42 and 50 of whole embryo and adult brain. Reverse transcription was performed from 1 g of total RNA, and 1/40 of the RT products was subjected to PCR using ³²P-dCTP. The PCR primers were designed in the common region between the intact and the truncated forms [5'-TCCCACAACAAACCTAAGTT-3' (SEQ ID NO: 12) and 5'-ATGTCCTCACTGGAAAGATC-3' (SEQ ID NO: 13)]. PCR for Histon H4 was also performed using the same templates as a control: [5'-CGGGATAACATTCAGGGTATCACT-3' ( SEQ ID NO: 14 ) and 5'-ATCCATGGCGGTAACTGTCTTCCT-3' (SEQ ID NO: 15)]. The number of cycles was 24 and 19 for Xreelin and Histon H4, respectively.

### Results

The time course of Xreelin expression at the transcription level was determined by RT-PCR (Fig. 4A). The primer set used in this assay was designed to amplify the common sequence between the intact form and the truncated form. To normalize each sample, PCR using a primer set for Histon H4 was performed. No Xreelin mRNA was detected during early development, and it first appeared in late neurula (st. 28). In tadpole stages, the signals became much stronger than those in neurula. Xreelin transcripts were also detected in the adult brain. In mouse development, Reelin mRNA becomes detectable after E8 by in situ hybridization and continues to be expressed in the adult brain (Ikeda, Y. & Terashima, T., 1997, Dev. Dyn. 210, 157-172; Schiffmann, S. N., Bernier, B. & Goffinet, A.M., 1997, European Journal of Neuroscience 9, 1055-1071.; Alcantara, S., Ruiz, M., D' Arcangelo, G., Ezan, F., de Lecea, L. & Curran, T., 1998, J. neurosci. 18, 7779-7799). Since the late neurula in Xenopus development is the switching point between neurulation and morphogenesis of the CNS, this stage corresponds to E8 of the mouse embryo in neural development. Therefore, the time course of Xreelin expression is similar to that of the mouse Reelin.

### Example 4 Quantification of the intact and the truncated form-mRNA

### Method

The amount of transcripts of the intact and truncated forms of Xreelin was evaluated by ABI PRISM 7700 (Perkin Elmer) using the gene-specific primers and oligonucleotide probes coupled with FAM (5-carboxyfluorescein) at the 5' end and TAMRA (N, N, N'-tetramethyl-5-carboxyrhodamine) at the 3' end (TaqMan probes). Total RNA was prepared from various stages (2-cell stage, st. 22, st. 28 and st. 43 whole embryos) and various parts of st. 50-54 brain (Fig. 5C). Reverse transcription (RT) was performed from 1 g of these RNA samples using Super Script II (Gibco BRL) and random hexamer, and 1/40 of the products was subjected to PCR using TaqMan probes. The procedure for this PCR was according to the protocol of TaqMan PCR Reagent Kit (Perkin Elmer). At each point, the averages and standard deviations were obtained by three independent PCR from one RT-product. To detect the intact form, one set of primers [5'-GTCCTGATCTACAAACACCTGCTACT-3' (SEQ ID NO: 16) and 5'-AGGTAGCACATGGACAAAATCC-3' (SEQ ID NO: 17)] and a TaqMan probe [5'-(FAM)CTGAAGCAAACCAGTCACCGTGGTCA(TAMRA)-3' (SEQ ID NO: 18)] were used. For the truncated form, the primer set [5'-TAGTGAGTGTGACAATCAGAAGTGA-3' (SEQ ID NO: 19)] and [5'-GGCCCTTTCTGGATAAGAATC-3' (SEQ ID NO: 20)], and a TaqMan probe [5'-(FAM)TCAACCATTTGCTCATACAGATGCACA(TAMRA)-3' (SEQ ID NO: 21)] were used. The copy numbers were estimated by a standard curve made from a dilution-series of plasmid DNA containing the intact form or the truncated form-specific sequence.

### Results

Then the developmental profile of the truncated in comparison with the intact form was examined. To address this issue, the amount of intact form and truncated form RNA was determined at several time-points (Fig. 4B). The amount of RNA was quantified by the TaqMan PCR technique using probes labeled with fluorescent dye, FAM and TAMRA, on the 5' and 3' ends, and then the degradation of the probe was observed during PCR. Expression of the truncated from is first observed around st. 28 and become much stronger in the later stages. This expression pattern resembles that of the intact form, and the proportion of the amount of truncated form versus intact form is constantly 5 to 10% throughout development. These results indicate that the expression profile of the both forms during development is regulated in the same way, although the absolute amounts differ.

### Example 5 Analysis of Xreelin mRNA Localization by In Situ Hybridization

### Method

The SDS-based in situ hybridization protocol was used (Shain, D.H. & Zuber, M.X., 1996, J. Biochem. Biophys. Methods 31, 185-188). Whole embryos of st. 35/36 were fixed in MEMFA buffer (Harland, R.M. in Methods in Cell biology, 1991, Academic Press Inc., San Diego, pp685-694) for 90 min. The brains of st.47 were dissected out in MEMFA and fixed in fresh MEMFA for 90 min. The digoxygenin labeled-RNA probe was synthesized by in vitro transcription using plasmid DNA containing the nucleotide sequence corresponding to 414-1252 in Fig. 1A as a template. The alkaline phosphatase chromogenic reaction was carried out in Purple AP (Boehringer-Manheim) for several hours at room temperature. The brain of st. 51 was dissected out in 4% paraformaldehyde in 70% PBS, and fixed in fresh same fixative overnight. The specimens embedded in OCT compound (Tissue Tech) were sectioned in 20 µm thick, and employed to in situ hybridization on the same way as for the whole embryos/brains with the exception that the NBT/BCIP solution in alkaline phosphatase buffer was used in the step of chromogenic reaction. RNA probes to the intact and truncated forms were synthesized from the sequences corresponded to 1296 to 1825 in Fig. 1A and 1302 to 2099 in fig. 1B, respectively. RNA probes to XdII and eomesodermin were prepared from the plasmid containing the PCR fragment obtained by using each gene-specific primers [5'-CCTCCAAGTCTGCCTTTATG-3' (SEQ ID NO: 22) and 5'-GCGGACAACAATATGCAAGG-3' (SEQ ID NO: 23)] for XdII, and [5'-GCGGACAACAATATGCAAGG-3' (SEQ ID NO: 24) and 5'-GGTTGTTGACAAACTGGTCC-3'(SEQ ID NO: 25)] for eomesodermin from the cDNA prepared from st. 51 Xenopus tadpole.

### Results

Reelin molecule is required for well-arranged lamination in the mouse brain development. Whereas the Reelin counterpart exists in Xenopus, its telencephalon shows no obvious laminated structure. Therefore, it is important whether or not Xreelin is expressed in the Xenopus dorsal pallium, which is a homologue of the mouse neocortex (Northcutt, G. R. & Kaas, J.H., 1995, Trends Neurosci 18, 373-379,; Fernandez, A. S., Pieau, C., Reperant, J., Boncinelli, E. & Wassf, M., 1998, Development 125, 2099-2111). To address this point, the distribution of Xreelin mRNA in telencephalon was examined more precisely. XdII is a Xenopus counterpart of distalless (Asano, M., Emori, Y., Saigo, K. & Shiokawa, K., 1992, J. Biol. Chem. 267, 5044-5047), and known to be expressed in the striatum (Asano, M., Emori, Y., Saigo, K. & Shiokawa, K., 1992, J. Biol. Chem. 267, 5044-5047). XdII mRNA is localized in the ventral side of telencephalon (Fig. 5D), and the Xreelin transcript is found more dorsally (lateral pallium) to the XdII-positive region (Fig. 5C). In the dorsal pallium, which is lying dorsally to the lateral pallium, Xreelin is expressed weakly in a few scattered cells near the surface of the telencephalic vesicle. These cells might correspond to the Cajal-Retzois cells of the mouse neocortex and have some function in the morphogenetic events other than the multi-layer alignment of neuroblasts.

In developing olfactory bulb of a mouse, the Reelin transcript is expressed in mitral cells. The Xreelin-expressing cells were identified in the olfactory bulb in Xenopus. Eomesodermin (Eomd) is known to be specifically expressed in mitral cells of the olfactory bulb (Ryan, K., Garett, N., Mitchell, A. & Gurdon, J. B., 1996, Cell 87, 989-1000; Ryan, K., Butler, K., Bellefroid, E. & Burdon, J. B., 1998, Mech. Dev. 75, 167-170). Using a probe to Eomd, it was clarified that the mitral cells are arranged in a "V" shaped pattern on horizontal sections of st. 51 olfactory bulb (Fig. 5F), and the Xreelin transcript is detected in the same pattern (Fig. 5E). In consequently, it is concluded that Xreelin is expressed in mitral cells in the developing olfactory bulb.

Whole mount in situ hybridization of brains revealed that Xreelin is expressed in the tectum (Fig. 5B). To elucidate the localization of Xreelin-expressing cells in detail, an Xreelin-specific probe was used to hybridize cross sections of st. 54 tectum. Xreelin was found beneath the stratum opticum, which is the most superficial layer of the tectum (Fig. 5B). The retinal axons coming through the stratum opticum turn into the underlying multiple cell-layers and terminate at a specific subset of lamina. So far, the distribution of mouse Reelin mRNA in the superior colliculus has not been reported in detail. However, homozygous Reeler mice show some abnormality in the retinotectal projection, whereas the cytoarchitecture itself is rather normal (Frost, D. O., Edwards, M.A., Sachs, G. M. & Caviness, V. J., 1986, Brain Res 393, 109-20). Taken together, these data suggest that Xreelin has some role in retinotectal projection during development.

In the spinal cord, Xreelin signals are found mainly in the medial to intermedio-lateral portions slightly ventral to the middle plane between the dorsal extremity and the ventral extremity. Weak signals are also detected in the dorsal horn (Fig. 5H). These expression patterns are similar to those in mice (Ikeda, Y. & Terashima, T., 1997, Dev. Dyn. 210, 157-172; Schiffmann, S. N., Bernier, B. & Goffinete, A. M., 1997, European Journal of Neuroscience 9, 1055-1071; Alcanara, S., Ruiz, M., D'Arcangelo, G., Ezan, F., De Lecea, L. & Curran, T., 1998, J. Neurosci. 18, 7779-7799).

To investigate the localization of the truncated form of Xreelin, in situ hybridization was carried out using a truncated form-specific probe. Thus, faint signals were detected in the cerebellum (Fig. 6B) in a similar pattern to the intact form (Fig. 6A), but no in situ signal was detected in other regions. Next, TaqMan PCR analysis was performed using RNA from various brain regions of Xenopus (Fig. 6C and 6D). A large amount of the intact form mRNA was found in the olfactory bulb, tectum and cerebellum, which was compatible with the expression pattern revealed by in situ hybridization. On the other hand, the truncated form of Xreelin was distributed in a similar manner to the intact form, and the ratio of the truncated form to the intact form was about 10% in each region. Considering the results obtained at various stages (Fig. 4B), it is likely that the expression profile of both forms is regulated by a common mechanism.

### Example 6 Existence of Truncated Reelin Protein in Mice

### Protocol

Mouse truncated Reelin protein was confirmed to exist by 3'-RACE. Total RNA was extracted from the spinal cord excised from a Reeler heterologous embryo on day 18 of the embryonal stage (the genotype had been specified by PCR). First strand synthesis using a primer [5'-GGCCACGCGTCGACTAGTACGAATTCATCTATAGC(T)₁₇-3' (SEQ ID NO: 9)], and then PCR using an Adaptor primer AP2 [5'-CGCGTCGACTAGTACGAATT-3' (SEQ ID NO: 26)] and a Reelin gene-specific primer RL-11 [5'-CTGATTGGATTCAGCTGGAG-3' (SEQ ID NO: 27)] were performed. Further, the PCR product was subjected to nested PCR using AP2 and a Reelin gene-specific primer RL-12 [5'-ATTCAGCCCACAGAGAAGTC-3' (SEQ ID NO: 28)].

### Results

Three bands were confirmed by the PCR. The bands were separately incorporated into pGEM-T Easy vectors, and sequenced. Thus, one of them was an alternative splicing product which contains upto exon14 of mouse Reelin followed by an unknown sequence. The difference between the alternative splicing product and a full length Reelin protein is replacement of the final amino acid of exon14, serine by arginine immediately followed by a termination codon. Therefore, the alternative splicing product was determined to be the truncated form. At the end of the 3'-untranslated region of the mouse truncated Reelin, multiple poly-adenylation signals (AATAAA) were confirmed.

The truncated Reelin protein of this invention and DNA encoding the protein can be used for treatment of diseases including agyria due to abnormal neuronal alignment.

## Claims

1. A truncated Reelin protein comprising an F-spondin domain and a CR-50 recognition site of a Reelin protein but containing no repeat site.

2. The truncated Reelin protein of claim 1 which is derived from Xenopus or mice.

3. The truncated Reelin protein of claim 2 which is either one of the following proteins (a) or (b):
(a) a protein consisting of an amino acid sequence represented by SEQ ID NO: 2, or
(b) a protein consisting of an amino acid sequence differing from the amino acid sequence of SEQ ID NO: 2 by deletion, substitution, or addition of one or more amino acids, and having Reelin protein activity.

4. The truncated Reelin protein of claim 2 which is either one of the following proteins (a) or (b):
(a) a protein consisting of an amino acid sequence represented by SEQ ID NO: 4, or
(b) a protein consisting of an amino acid sequence differing from the amino acid sequence of SEQ ID NO: 4 by deletion, substitution, or addition of one or more amino acids, and having Reelin protein activity.

5. A DNA encoding a truncated Reelin protein comprising an F-spondin domain and a CR-50 recognition site of a Reelin protein but containing no repeat site.

6. The DNA of claim 5 which is derived from Xenopus or mice.

7. The DNA of claim 6 encoding either one of the following truncated Reelin proteins (a) or (b):
(a) a protein consisting of an amino acid sequence represented by SEQ ID NO: 2, or
(b) a protein consisting of an amino acid sequence differing from the amino acid sequence of SEQ ID NO: 2 by deletion, substitution, or addition of one or more amino acids, and having Reelin protein activity.

8. The DNA of claim 7, which is any one of the following DNAs (a) to (c):
(a) a DNA having a nucleotide sequence represented by SEQ ID NO: 1,
(b) a DNA hybridizing to a nucleic acid probe which comprises a sequence of 1456^{th} to 2273^{rd} nucleotide in the nucleotide sequence of SEQ ID NO: 1 under stringent conditions, and encoding a protein with Reelin protein activity, or
(c) a DNA having a nucleotide sequence which is a degenerate sequence of that of the DNA (a) or (b).

9. The DNA of claim 6 encoding a truncated Reelin protein which is either one of the following proteins (a) or (b):
(a) a protein consisting of an amino acid sequence represented by SEQ ID NO: 4, or
(b) a protein consisting of an amino acid sequence differing from the amino acid sequence of SEQ ID NO: 4 by deletion, substitution, or addition of one or more amino acids, and having Reelin protein activity.

10. The DNA of claim 9 which is any one of the following DNAs (a) to (c):
(a) a DNA having a nucleotide sequence represented by SEQ ID NO: 3,
(b) a DNA hybridizing to a nucleic acid probe which comprises a sequence of 2053^{rd} to 2758^{th} nucleotide in the nucleotide sequence of SEQ ID NO: 3, and encoding a protein with Reelin protein activity, or
(c) a DNA having a nucleotide sequence which is a degenerate sequence of that of the DNA (a) or (b).


SEQUENCE LISTING

<110> RIKEN

<120> Truncated Reelin Protein and DNA Encoding the Same

<130> AHB/FP5925169

<140>
<141>

<150> JP 2000-109954
<151> 2000-04-11

<160> 28

<170> PatentIn Ver. 2.0

<210> 1
<211> 2274
<212> DNA
<213> Xenopus laevis

<220>
<221> CDS
<222> (157)..(1455)

<220>
<221> sig_peptide

<222> (157)..(234)

<220>
<221> misc_feature
<222> (241)..(726)
<223> F-spondin domain

<220>
<221> misc_feature
<222> (847)..(1197)
<223> CR-50 epitope region

<400> 1
cattctactg tcacgttaac tttccatttt cttcacttta actttgaaga atttaaaaaa 60

aaccattaat tatatattta tataaatata tatatataan ctctgtatcc caggctgctt 120

atgaagaaag ctcattaaga acagtgggac ccagga atg gaa ctg ctc cac acc 174
Met Glu Leu Leu His Thr
1               5

ttc tgc ggt ggg cgc tgg act ttg ctg ctc ttc acg ggg atc ttg tgc 222
Phe Cys Gly Gly Arg Trp Thr Leu Leu Leu Phe Thr Gly Ile Leu Cys
            10                  15                  20

ttt gtt gtt gcc cgc gga gtg ggg tat tat ccc agg ttc tct cca ttc 270
Phe Val Val Ala Arg Gly Val Gly Tyr Tyr Pro Arg Phe Ser Pro Phe
        25                  30                  35

ttt ttc ctt tgc act cat cat gga gaa ctg gaa gga gat ggg gaa caa 318
Phe Phe Leu Cys Thr His His Gly Glu Leu Glu Gly Asp Gly Glu Gln
    40                  45                  50

gga gaa gtg ctc atc tct ctg cac ctg gcg ggc aac ccc agc tac tac 366
Gly Glu Val Leu Ile Ser Leu His Leu Ala Gly Asn Pro Ser Tyr Tyr
55                  60                  65                  70

ata cct ggg cag gag tac cat gtg acc ata tcc act agt acc ttc ttt 414
Ile Pro Gly Gln Glu Tyr His Val Thr Ile Ser Thr Ser Thr Phe Phe
                75                  80                  85

gat ggt ctt ctg gtg act gga ctt tac act tct acc agt gtt caa gcg 462
Asp Gly Leu Leu Val Thr Gly Leu Tyr Thr Ser Thr Ser Val Gln Ala
            90                  95                  100

tct cag agc att gga ggc tct aaa gca ttt gga ttt ggt att atg agc 510
Ser Gln Ser Ile Gly Gly Ser Lys Ala Phe Gly Phe Gly Ile Met Ser
        105                 110                 115

gac cgt cag ttt ggt acc cag ttt atg tgc agt gtc gtt gct tcc cac 558
Asp Arg Gln Phe Gly Thr Gln Phe Met Cys Ser Val Val Ala Ser His
    120                 125                 130

gtg agt cat ctt ccc aca aca aac cta agt ttt gta tgg att gca cca 606
Val Ser His Leu Pro Thr Thr Asn Leu Ser Phe Val Trp Ile Ala Pro
135                 140                 145                 150

cca gca ggt aca gga tgt gtc aac ttc atg gcc aca gca aca cat agg 654

Pro Ala Gly Thr Gly Cys Val Asn Phe Met Ala Thr Ala Thr His Arg
                155                 160                 165

gga caa gtt att ttc aag gat gcc ctg gca caa caa ctg tgc gaa caa 702
Gly Gln Val Ile Phe Lys Asp Ala Leu Ala Gln Gln Leu Cys Glu Gln
            170                 175                 180

gga gct cct act gaa gct ccc ttg cgg cct aat tta gcc gaa att cac 750
Gly Ala Pro Thr Glu Ala Pro Leu Arg Pro Asn Leu Ala Glu Ile His
        185                 190                 195

agt gaa agc atc ctt tta cga gat gat ttt gac tca tat aag ctt cag 798
Ser Glu Ser Ile Leu Leu Arg Asp Asp Phe Asp Ser Tyr Lys Leu Gln
    200                 205                 210

gaa ttg aat cca aat att tgg ctc cag tgc aga aat tgc gaa gtt ggt 846
Glu Leu Asn Pro Asn Ile Trp Leu Gln Cys Arg Asn Cys Glu Val Gly
215                 220                 225                 230

gag cag tgt ggt gca att atg cat ggt ggg gca gtc act ttt tgt gat 894
Glu Gln Cys Gly Ala Ile Met His Gly Gly Ala Val Thr Phe Cys Asp
                235                 240                 245

ccg tat gga cca aga gaa ttg ata act gtt caa atg aac aca act acg 942
Pro Tyr Gly Pro Arg Glu Leu Ile Thr Val Gln Met Asn Thr Thr Thr
            250                 255                 260

gca tct gtt ttg cag ttt tct att ggg tca gga tcg tgc agg ttc agc 990
Ala Ser Val Leu Gln Phe Ser Ile Gly Ser Gly Ser Cys Arg Phe Ser

        265                 270                 275

tat tca gac cct gga att gtg gtg tca tac aca aag aat aat tca tct 1038
Tyr Ser Asp Pro Gly Ile Val Val Ser Tyr Thr Lys Asn Asn Ser Ser
    280                 285                 290

agt tgg atg cca ttg gag aga att agt gct cct tcc aat gtt agc acc 1086
Ser Trp Met Pro Leu Glu Arg Ile Ser Ala Pro Ser Asn Val Ser Thr
295                 300                 305                 310

atc att cac att att tac cta cct cct gaa gct aaa gga gaa aat gtg 1134
Ile Ile His Ile Ile Tyr Leu Pro Pro Glu Ala Lys Gly Glu Asn Val
                315                 320                 325

aaa ttc cgt tgg agg cag gag aac atg cag gca ggt gat gtg tat gaa 1182
Lys Phe Arg Trp Arg Gln Glu Asn Met Gln Ala Gly Asp Val Tyr Glu
            330                 335                 340

gcc tgc tgg gca ctg gat aac att ttg att atc aat gct gct cat aaa 1230
Ala Cys Trp Ala Leu Asp Asn Ile Leu Ile Ile Asn Ala Ala His Lys
        345                 350                 355

gaa gtc gtg tta gaa gac aat cta gat cca atg gac aca gga aac tgg 1278
Glu Val Val Leu Glu Asp Asn Leu Asp Pro Met Asp Thr Gly Asn Trp
    360                 365                 370

ctt ttt ttc cct ggg gct act gta aag cat acc tgt cag tcg gat gga 1326
Leu Phe Phe Pro Gly Ala Thr Val Lys His Thr Cys Gln Ser Asp Gly
375                 380                 385                 390

aac tct ata tat ttt cat ggt aca gaa agc agt gaa tac aac ttt gct 1374
Asn Ser Ile Tyr Phe His Gly Thr Glu Ser Ser Glu Tyr Asn Phe Ala
                395                 400                 405

act acc aga gat gtg gat ctt tcc agt gag gac atc cag gac cag tgg 1422
Thr Thr Arg Asp Val Asp Leu Ser Ser Glu Asp Ile Gln Asp Gln Trp
            410                 415                 420

tct gaa gag ttt gag aat cta cca gct ggg taa atttagatg tagccatgag 1475
Ser Glu Glu Phe Glu Asn Leu Pro Ala Gly
        425                 430

cattacattt tatcacgtga aaatgcaaga aacagtattt atatacatat tttaaaggtc 1535

aatacagaac cctataaatg gcaggttagg gctaccatgt aaatattttt aatgttcata 1595

atgtcatagg tggtaagtat tttacatagc agttactgat tgattattat tgtttgtctt 1655

ttacccagtt acagctaaca cacagggcat ttttttccaa tggcaacatc cattttgccg 1715

ctctgagcag aacatttgtt tcatttatgg catttgaacc tgtgtctatg agagtgcagc 1775

taaaataaac ttcctggcta tgggtgttac catacaacac tggtacctca tgacatatga 1835

aaaatatgac tcacattaaa tcagtaagat cagttcaagt atagtacggt gcattaatct 1895

gccaataaac atttagaatt gtattttata ttttatattt aagattagaa ttgactccat 1955

tcttgtacct tgcatcacat ttgtggctag tttatgggtc aatagacagc catcatacat 2015

tagtcagagt aaatcgagca ttacaaaact caatgagcca tagtgagtgt gacaatcaga 2075

agtgactgtc aagtaaatca accatttgct catacagatg cacatttgaa cagtggattc 2135

ttatccagaa agggccattt tttactatca ctctgggatt taaatgccac ttctaattgg 2195

aacttccagg tcacaaaaat agaatggaca tttaaacatc atggttctca ttacccctaa 2255

taaaactccg gtttttttta 2274

<210> 2
<211> 432
<212> PRT
<213> Xenopus laevis

<400> 2
Met Glu Leu Leu His Thr Phe Cys Gly Gly Arg Trp Thr Leu Leu Leu
1               5                   10                  15

Phe Thr Gly Ile Leu Cys Phe Val Val Ala Arg Gly Val Gly Tyr Tyr
            20                  25                  30

Pro Arg Phe Ser Pro Phe Phe Phe Leu Cys Thr His His Gly Glu Leu
        35                  40                  45

Glu Gly Asp Gly Glu Gln Gly Glu Val Leu Ile Ser Leu His Leu Ala

50                  55                  60

Gly Asn Pro Ser Tyr Tyr Ile Pro Gly Gln Glu Tyr His Val Thr Ile
65                  70                  75                  80

Ser Thr Ser Thr Phe Phe Asp Gly Leu Leu Val Thr Gly Leu Tyr Thr
                85                  90                  95

Ser Thr Ser Val Gln Ala Ser Gln Ser Ile Gly Gly Ser Lys Ala Phe
            100                 105                 110

Gly Phe Gly Ile Met Ser Asp Arg Gln Phe Gly Thr Gln Phe Met Cys
        115                 120                 125

Ser Val Val Ala Ser His Val Ser His Leu Pro Thr Thr Asn Leu Ser
    130                 135                 140

Phe Val Trp Ile Ala Pro Pro Ala Gly Thr Gly Cys Val Asn Phe Met
145                 150                 155                 160

Ala Thr Ala Thr His Arg Gly Gln Val Ile Phe Lys Asp Ala Leu Ala
                165                 170                 175

Gln Gln Leu Cys Glu Gln Gly Ala Pro Thr Glu Ala Pro Leu Arg Pro
            180                 185                 190

Asn Leu Ala Glu Ile His Ser Glu Ser Ile Leu Leu Arg Asp Asp Phe
        195                 200                 205

Asp Ser Tyr Lys Leu Gln Glu Leu Asn Pro Asn Ile Trp Leu Gln Cys
    210                 215                 220

Arg Asn Cys Glu Val Gly Glu Gln Cys Gly Ala Ile Met His Gly Gly
225                 230                 235                 240

Ala Val Thr Phe Cys Asp Pro Tyr Gly Pro Arg Glu Leu Ile Thr Val
                245                 250                 255

Gln Met Asn Thr Thr Thr Ala Ser Val Leu Gln Phe Ser Ile Gly Ser
            260                 265                 270

Gly Ser Cys Arg Phe Ser Tyr Ser Asp Pro Gly Ile Val Val Ser Tyr
        275                 280                 285

Thr Lys Asn Asn Ser Ser Ser Trp Met Pro Leu Glu Arg Ile Ser Ala
    290                 295                 300

Pro Ser Asn Val Ser Thr Ile Ile His Ile Ile Tyr Leu Pro Pro Glu
305                 310                 315                 320

Ala Lys Gly Glu Asn Val Lys Phe Arg Trp Arg Gln Glu Asn Met Gln
                325                 330                 335

Ala Gly Asp Val Tyr Glu Ala Cys Trp Ala Leu Asp Asn Ile Leu Ile
            340                 345                 350

Ile Asn Ala Ala His Lys Glu Val Val Leu Glu Asp Asn Leu Asp Pro
        355                 360                 365

Met Asp Thr Gly Asn Trp Leu Phe Phe Pro Gly Ala Thr Val Lys His
    370                 375                 380

Thr Cys Gln Ser Asp Gly Asn Ser Ile Tyr Phe His Gly Thr Glu Ser
385                 390                 395                 400

Ser Glu Tyr Asn Phe Ala Thr Thr Arg Asp Val Asp Leu Ser Ser Glu
                405                 410                 415

Asp Ile Gln Asp Gln Trp Ser Glu Glu Phe Glu Asn Leu Pro Ala Gly
            420                 425                 430

<210> 3
<211> 2745
<212> DNA
<213> Mus musculus

<220>
<221> CDS
<222> (283)..(2052)

<220>
<221> sig_peptide
<222> (283)..(363)

<220>
<221> misc_feature

<222> (284)..(849)
<223> F-spondin domain

<220>
<221> misc_feature
<222> (970)..(1320)
<223> CR-50 epitope region

<400> 3
ggggcgtcgc gtgcacaccg gcggcggcgg cgctcggagg cggacgacgc gctctcggcg 60

cccgcggccc cggttccccc cgcgctctcg ctccggcggc ccaaagtaac ttcgggagcc 120

tcggtctccc gctaacttcc ccccgcgggc tcggttgccc ggacccgctc ggctcgagcc 180

cgccgccggc tcgccttccc cgcacgcggc tcctccgtgc cggtgcctcc gaaagtggat 240

gagagagcgc gcggggcgcg cggcggcacg gagcgcggcg gc atg gag cgc ggc 294
Met Glu Arg Gly
1

tgc tgg gcg ccg cgg gct ctc gtc ctg gcc gtg ctg ctg ctg ctg gcg 342
Cys Trp Ala Pro Arg Ala Leu Val Leu Ala Val Leu Leu Leu Leu Ala
5                   10                  15                  20

acg ctg agg gcg cgc gcg gcc acc ggc tac tac ccg cgc ttc tcg cct 390
Thr Leu Arg Ala Arg Ala Ala Thr Gly Tyr Tyr Pro Arg Phe Ser Pro
                25                  30                  35

ttc ttt ttc ctg tgc acc cac cac ggg gag ctg gaa ggg gat ggg gag 438
Phe Phe Phe Leu Cys Thr His His Gly Glu Leu Glu Gly Asp Gly Glu
            40                  45                  50

cag ggc gag gtg ctc att tcc ctg cac att gcg ggc aac ccc acc tac 486
Gln Gly Glu Val Leu Ile Ser Leu His Ile Ala Gly Asn Pro Thr Tyr
        55                  60                  65

tac gta ccg gga cag gaa tac cat gtt aca att tca aca agc acc ttc 534
Tyr Val Pro Gly Gln Glu Tyr His Val Thr Ile Ser Thr Ser Thr Phe
    70                  75                  80

ttt gat ggc ttg ctg gtg acg gga ctc tat acc tcg aca agc atc cag 582
Phe Asp Gly Leu Leu Val Thr Gly Leu Tyr Thr Ser Thr Ser Ile Gln
85                  90                  95                  100

tct tct cag agc att gga ggc tcc agc gcc ttt gga ttc ggg atc atg 630
Ser Ser Gln Ser Ile Gly Gly Ser Ser Ala Phe Gly Phe Gly Ile Met
                105                 110                 115

tcc gac cac cag ttt ggt aac cag ttt atg tgc agt gtg gtg gcc tct 678
Ser Asp His Gln Phe Gly Asn Gln Phe Met Cys Ser Val Val Ala Ser
            120                 125                 130

cat gtg agt cac ctg cct aca acc aac ctc agc ttt gtc tgg att gcc 726
His Val Ser His Leu Pro Thr Thr Asn Leu Ser Phe Val Trp Ile Ala
        135                 140                 145

cca cca gct ggc aca ggc tgt gtg aat ttc atg gct act gca aca cat 774

Pro Pro Ala Gly Thr Gly Cys Val Asn Phe Met Ala Thr Ala Thr His
    150                 155                 160

agg ggc cag gtg att ttc aaa gac gca ctg gcc cag cag ctg tgt gaa 822
Arg Gly Gln Val Ile Phe Lys Asp Ala Leu Ala Gln Gln Leu Cys Glu
165                 170                 175                 180

caa gga gct ccc aca gag gcc act gct tac tcg cac ctt gct gaa ata 870
Gln Gly Ala Pro Thr Glu Ala Thr Ala Tyr Ser His Leu Ala Glu Ile
                185                 190                 195

cac agt gac agt gtg atc cta cga gat gac ttt gac tcc tac cag caa 918
His Ser Asp Ser Val Ile Leu Arg Asp Asp Phe Asp Ser Tyr Gln Gln
            200                 205                 210

ctg gaa ttg aac ccc aac ata tgg gtt gaa tgc agc aac tgt gag atg 966
Leu Glu Leu Asn Pro Asn Ile Trp Val Glu Cys Ser Asn Cys Glu Met
        215                 220                 225

gga gag cag tgt ggc acc atc atg cat ggc aat gct gtc acc ttc tgt 1014
Gly Glu Gln Cys Gly Thr Ile Met His Gly Asn Ala Val Thr Phe Cys
    230                 235                 240

gag ccg tac ggc cct cga gag ctg acc acc aca tgc ctg aac aca aca 1062
Glu Pro Tyr Gly Pro Arg Glu Leu Thr Thr Thr Cys Leu Asn Thr Thr
245                 250                 255                 260

aca gca tct gtc ctc cag ttt tcc att ggg tca gga tca tgt cga ttt 1110
Thr Ala Ser Val Leu Gln Phe Ser Ile Gly Ser Gly Ser Cys Arg Phe

                265                 270                 275

agt tac tct gac ccc agc atc act gtg tca tac gcc aag aac aat acc 1158
Ser Tyr Ser Asp Pro Ser Ile Thr Val Ser Tyr Ala Lys Asn Asn Thr
            280                 285                 290

gct gat tgg att cag ctg gag aaa att aga gcc cct tcc aat gtg agc 1206
Ala Asp Trp Ile Gln Leu Glu Lys Ile Arg Ala Pro Ser Asn Val Ser
        295                 300                 305

aca gtc atc cac atc ctg tac ctc ccc gag gaa gcc aaa ggg gag agc 1254
Thr Val Ile His Ile Leu Tyr Leu Pro Glu Glu Ala Lys Gly Glu Ser
    310                 315                 320

gtg cag ttc cag tgg aaa cag gac agc ctg cga gtg ggt gag gtg tat 1302
Val Gln Phe Gln Trp Lys Gln Asp Ser Leu Arg Val Gly Glu Val Tyr
325                 330                 335                 340

gag gcc tgc tgg gcc ctg gat aac atc ctg gtc atc aat tca gcc cac 1350
Glu Ala Cys Trp Ala Leu Asp Asn Ile Leu Val Ile Asn Ser Ala His
                345                 350                 355

aga gaa gtc gtt ctg gag gac aac ctc gac ccg gtc gac acg ggc aac 1398
Arg Glu Val Val Leu Glu Asp Asn Leu Asp Pro Val Asp Thr Gly Asn
            360                 365                 370

tgg ctc ttc ttc cct gga gca acg gtc aag cat agc tgt cag tca gat 1446
Trp Leu Phe Phe Pro Gly Ala Thr Val Lys His Ser Cys Gln Ser Asp
        375                 380                 385

ggg aac tcc att tat ttc cat gga aat gaa ggc agc gag ttc aat ttt 1494
Gly Asn Ser Ile Tyr Phe His Gly Asn Glu Gly Ser Glu Phe Asn Phe
    390                 395                 400

gcc acc acc cgg gat gta gat ctt tct aca gag gat att caa gag cag 1542
Ala Thr Thr Arg Asp Val Asp Leu Ser Thr Glu Asp Ile Gln Glu Gln
405                 410                 415                 420

tgg tca gaa gaa ttt gag agc cag ccc aca gga tgg gat atc ttg gga 1590
Trp Ser Glu Glu Phe Glu Ser Gln Pro Thr Gly Trp Asp Ile Leu Gly
                425                 430                 435

gca gta gtt ggt gca gac tgt gga acc gta gaa tca gga cta tca ctg 1638
Ala Val Val Gly Ala Asp Cys Gly Thr Val Glu Ser Gly Leu Ser Leu
            440                 445                 450

gtg ttc ctc aaa gat gga gag agg aag ctt tgc acc ccc tac atg gat 1686
Val Phe Leu Lys Asp Gly Glu Arg Lys Leu Cys Thr Pro Tyr Met Asp
        455                 460                 465

aca act ggt tat ggc aac ctg agg ttc tac ttc gtt atg gga gga atc 1734
Thr Thr Gly Tyr Gly Asn Leu Arg Phe Tyr Phe Val Met Gly Gly Ile
    470                 475                 480

tgt gac cct gga gtc tct cat gaa aac gat atc atc tta tat gca aag 1782
Cys Asp Pro Gly Val Ser His Glu Asn Asp Ile Ile Leu Tyr Ala Lys
485                 490                 495                 500

att gaa gga aga aaa gaa cac att gca ctg gac act ctt acc tat tct 1830
Ile Glu Gly Arg Lys Glu His Ile Ala Leu Asp Thr Leu Thr Tyr Ser
                505                 510                 515

tcc tat aag gtt ccg tct ttg gtt tct gtg gtc atc aac cct gaa ctt 1878
Ser Tyr Lys Val Pro Ser Leu Val Ser Val Val Ile Asn Pro Glu Leu
            520                 525                 530

cag aca cct gcc acc aaa ttt tgt ctc agg caa aag agc cac caa ggg 1926
Gln Thr Pro Ala Thr Lys Phe Cys Leu Arg Gln Lys Ser His Gln Gly
        535                 540                 545

tat aat cgg aat gtc tgg gct gtg gac ttc ttc cat gtg ctg ccc gtt 1974
Tyr Asn Arg Asn Val Trp Ala Val Asp Phe Phe His Val Leu Pro Val
    550                 555                 560

ctc cct tca aca atg tct cac atg atc cag ttt tct att aat ttg gga 2022
Leu Pro Ser Thr Met Ser His Met Ile Gln Phe Ser Ile Asn Leu Gly
565                 570                 575                 580

tgc ggc aca cac cag cct ggg aac agg tga gaagcatgcc gagtgtccta 2072
Cys Gly Thr His Gln Pro Gly Asn Arg
585 590

acatggtagg aaataaacac atgcactgga ccattgaagt aagtttgtca gtaggatttt 2132

tggatgggat tttaacaaaa tatccattaa gaaaatacag attcctactc cctccctaaa 2192

agagttcttt ggttaataaa tagaagggat gtgactgggt agatttttag gttagaatag 2252

tttcattcag ggagcttgat acaagttatc agaggtgttc accatgctgt gtggcagcat 2312

cccccgttct aacagattgc tgggtgaaga tgactgaaga caagattggc ttctgttggc 2372

tggtgacccc ttataatagg tatggaagtc aattagcact tcaagggcta tgacttctct 2432

gctcctcttg cataagtgtt gctcccatcc tctgtaaaga actttgctga cctcacattc 2492

acaggatgaa gtgacagtgt gagacatggt aattgcctag ctatctatca aattcaagag 2552

cacaaaccca gtttactgtg tattgtcctt cagacgtagc ttttatggca gtaatccaat 2612

ggcttgccct ctgaaggctg gtcaggcttc agtgagagat gacacattta gtaaaggtct 2672

tagagaaatc ccacattcat cgactcattc aaggtattta gctagaaata aaaagaatca 2732

aaaaaataaa tta 2745

<210> 4

<211> 589

<212> PRT

<213> Mus musculus

<400> 4

Met Glu Arg Gly Cys Trp Ala Pro Arg Ala Leu Val Leu Ala Val Leu

1               5                   10                  15

Leu Leu Leu Ala Thr Leu Arg Ala Arg Ala Ala Thr Gly Tyr Tyr Pro
            20                  25                  30
Arg Phe Ser Pro Phe Phe Phe Leu Cys Thr His His Gly Glu Leu Glu
        35                  40                  45
Gly Asp Gly Glu Gln Gly Glu Val Leu Ile Ser Leu His Ile Ala Gly
    50                  55                  60
Asn Pro Thr Tyr Tyr Val Pro Gly Gln Glu Tyr His Val Thr Ile Ser
65                  70                  75                  80
Thr Ser Thr Phe Phe Asp Gly Leu Leu Val Thr Gly Leu Tyr Thr Ser
                85                  90                  95
Thr Ser Ile Gln Ser Ser Gln Ser Ile Gly Gly Ser Ser Ala Phe Gly
            100                 105                 110
Phe Gly Ile Met Ser Asp His Gln Phe Gly Asn Gln Phe Met Cys Ser
        115                 120                 125
Val Val Ala Ser His Val Ser His Leu Pro Thr Thr Asn Leu Ser Phe
    130                 135                 140
Val Trp Ile Ala Pro Pro Ala Gly Thr Gly Cys Val Asn Phe Met Ala
145                 150                 155                 160
Thr Ala Thr His Arg Gly Gln Val Ile Phe Lys Asp Ala Leu Ala Gln
                165                 170                 175

Gln Leu Cys Glu Gln Gly Ala Pro Thr Glu Ala Thr Ala Tyr Ser His
            180                 185                 190
Leu Ala Glu Ile His Ser Asp Ser Val Ile Leu Arg Asp Asp Phe Asp
        195                 200                 205
Ser Tyr Gln Gln Leu Glu Leu Asn Pro Asn Ile Trp Val Glu Cys Ser
    210                 215                 220
Asn Cys Glu Met Gly Glu Gln Cys Gly Thr Ile Met His Gly Asn Ala
225                 230                 235                 240
Val Thr Phe Cys Glu Pro Tyr Gly Pro Arg Glu Leu Thr Thr Thr Cys
                245                 250                 255
Leu Asn Thr Thr Thr Ala Ser Val Leu Gln Phe Ser Ile Gly Ser Gly
            260                 265                 270
Ser Cys Arg Phe Ser Tyr Ser Asp Pro Ser Ile Thr Val Ser Tyr Ala
        275                 280                 285
Lys Asn Asn Thr Ala Asp Trp Ile Gln Leu Glu Lys Ile Arg Ala Pro
    290                 295                 300
Ser Asn Val Ser Thr Val Ile His Ile Leu Tyr Leu Pro Glu Glu Ala
305                 310                 315                 320
Lys Gly Glu Ser Val Gln Phe Gln Trp Lys Gln Asp Ser Leu Arg Val

                325                 330                 335

Gly Glu Val Tyr Glu Ala Cys Trp Ala Leu Asp Asn Ile Leu Val Ile

            340                 345                 350

Asn Ser Ala His Arg Glu Val Val Leu Glu Asp Asn Leu Asp Pro Val

        355                 360                 365

Asp Thr Gly Asn Trp Leu Phe Phe Pro Gly Ala Thr Val Lys His Ser

    370                 375                 380

Cys Gln Ser Asp Gly Asn Ser Ile Tyr Phe His Gly Asn Glu Gly Ser

385                 390                 395                 400

Glu Phe Asn Phe Ala Thr Thr Arg Asp Val Asp Leu Ser Thr Glu Asp

                405                 410                 415

Ile Gln Glu Gln Trp Ser Glu Glu Phe Glu Ser Gln Pro Thr Gly Trp

            420                 425                 430

Asp Ile Leu Gly Ala Val Val Gly Ala Asp Cys Gly Thr Val Glu Ser

        435                 440                 445

Gly Leu Ser Leu Val Phe Leu Lys Asp Gly Glu Arg Lys Leu Cys Thr

    450                 455                 460

Pro Tyr Met Asp Thr Thr Gly Tyr Gly Asn Leu Arg Phe Tyr Phe Val

465                 470                 475                 480

Met Gly Gly Ile Cys Asp Pro Gly Val Ser His Glu Asn Asp Ile Ile
                485                 490                 495
Leu Tyr Ala Lys Ile Glu Gly Arg Lys Glu His Ile Ala Leu Asp Thr
            500                 505                 510
Leu Thr Tyr Ser Ser Tyr Lys Val Pro Ser Leu Val Ser Val Val Ile
        515                 520                 525
Asn Pro Glu Leu Gln Thr Pro Ala Thr Lys Phe Cys Leu Arg Gln Lys
    530                 535                 540
Ser His Gln Gly Tyr Asn Arg Asn Val Trp Ala Val Asp Phe Phe His
545                 550                 555                 560
Val Leu Pro Val Leu Pro Ser Thr Met Ser His Met Ile Gln Phe Ser
                565                 570                 575
Ile Asn Leu Gly Cys Gly Thr His Gln Pro Gly Asn Arg
            580                 585

<210> 5
<211> 22
<212> DNA
<213> Artificial Sequence

<220>
<223> Description of Artificial Sequence:synthetic DNA

<220>

<221> modified_base

<222> (8)

<223> i

<400> 5

arttyggnaa ycarttyatg tg                                                22

<210> 6

<211> 16

<212> DNA

<213> Artificial Sequence

<220>

<223> Description of Artificial Sequence:synthetic DNA

<400> 6

tgytccccat ycartt                                                       16

<210> 7

<211> 20

<212> DNA

<213> Artificial Sequence

<220>

<223> Description of Artificial Sequence:synthetic DNA

<400> 7

atgtcctcac tggaaagatc                                                    20

<210> 8

<211> 20

<212> DNA

<213> Artificial Sequence

<220>

<223> Description of Artificial Sequence:synthetic DNA

<400> 8

cagcaacaca taggggacaa                                                    20

<210> 9

<211> 51

<212> DNA

<213> Artificial Sequence

<220>

<223> Description of Artificial Sequence:synthetic DNA

<400> 9

ggccacgcgt cgactagtac gaattcatct atagcttttt tttttttttt t                 51

<210> 10

<211> 35

<212> DNA

<213> Artificial Sequence

<220>

<223> Description of Artificial Sequence:synthetic DNA

<400> 10

cagtgtcgtt gcttcccacg tgagtcatct tccca 35

<210> 11

<211> 35

<212> DNA

<213> Artificial Sequence

<220>

<223> Description of Artificial Sequence:synthetic DNA

<400> 11

cgacaggtac aggatgtgtc aacttcatgg ccaca 35

<210> 12

<211> 20

<212> DNA

<213> Artificial Sequence

<220>

<223> Description of Artificial Sequence:synthetic DNA

<400> 12

tcccacaaca aacctaagtt 20

<210> 13

<211> 20

<212> DNA

<213> Artificial Sequence

<220>

<223> Description of Artificial Sequence:synthetic DNA

<400> 13

atgtcctcac tggaaagatc 20

<210> 14

<211> 24

<212> DNA

<213> Artificial Sequence

<220>

<223> Description of Artificial Sequence:synthetic DNA

<400> 14

cgggataaca ttcagggtat cact 24

<210> 15
<211> 24
<212> DNA
<213> Artificial Sequence

<220>
<223> Description of Artificial Sequence:synthetic DNA

<400> 15
atccatggcg gtaactgtct tcct                                              24

<210> 16
<211> 26
<212> DNA
<213> Artificial Sequence

<220>
<223> Description of Artificial Sequence:synthetic DNA

<400> 16
gtcctgatct acaaacacct gctact                                            26

<210> 17
<211> 22
<212> DNA

<213> Artificial Sequence

<220>
<223> Description of Artificial Sequence:synthetic DNA

<400> 17
aggtagcaca tggacaaaat cc                                                22

<210> 18
<211> 26
<212> DNA
<213> Artificial Sequence

<220>
<223> Description of Artificial Sequence:synthetic DNA

<400> 18
ctgaagcaaa ccagtcaccg tggtca                                            26

<210> 19
<211> 25
<212> DNA
<213> Artificial Sequence

<220>
<223> Description of Artificial Sequence:synthetic DNA

<400> 19
tagtgagtgt gacaatcaga agtga                                                25

<210> 20
<211> 21
<212> DNA
<213> Artificial Sequence

<220>
<223> Description of Artificial Sequence:synthetic DNA

<400> 20
ggccctttct ggataagaat c                                                    21

<210> 21
<211> 27
<212> DNA
<213> Artificial Sequence

<220>
<223> Description of Artificial Sequence:synthetic DNA

<400> 21
tcaaccattt gctcatacag atgcaca                                              27

<210> 22

<211> 20

<212> DNA

<213> Artificial Sequence

<220>

<223> Description of Artificial Sequence:synthetic DNA

<400> 22

cctccaagtc tgcctttatg                                                    20

<210> 23

<211> 20

<212> DNA

<213> Artificial Sequence

<220>

<223> Description of Artificial Sequence:synthetic DNA

<400> 23

gcggacaaca atatgcaagg                                                    20

<210> 24

<211> 20

<212> DNA

<213> Artificial Sequence

<220>

<223> Description of Artificial Sequence:synthetic DNA

<400> 24
gcggacaaca atatgcaagg                                                    20

<210> 25
<211> 20
<212> DNA
<213> Artificial Sequence

<220>
<223> Description of Artificial Sequence:synthetic DNA

<400> 25
ggttgttgac aaactggtcc                                                    20

<210> 26
<211> 20
<212> DNA
<213> Artificial Sequence

<220>
<223> Description of Artificial Sequence:synthetic DNA

<400> 26
cgcgtcgact agtacgaatt                                                    20

<210> 27

<211> 20

<212> DNA

<213> Artificial Sequence

<220>

<223> Description of Artificial Sequence:synthetic DNA

<400> 27

ctgattggat tcagctggag 20

<210> 28

<211> 20

<212> DNA

<213> Artificial Sequence

<220>

<223> Description of Artificial Sequence:synthetic DNA

<400> 28

attcagccca cagagaagtc 20